(19) Europäisches Patentamt
European Patent Office
Office européen des brevets

(11) Veröffentlichungsnummer : **0 307 358 B1**

(12) # EUROPÄISCHE PATENTSCHRIFT

(45) Veröffentlichungstag der Patentschrift : **12.02.92 Patentblatt 92/07**

(51) Int. Cl.$^5$ : **C07C 323/22,** C07C 69/738, C07C 49/175, C08K 5/07, C08K 5/37, C08L 27/06

(21) Anmeldenummer : **88810602.8**

(22) Anmeldetag : **02.09.88**

(54) **Etherhaltige oder thioetherhaltige 1,3-Diketone und deren Verwendung als Stabilisatoren für chlorhaltige Polymerisate.**

Die Akte enthält technische Angaben, die nach dem Eingang der Anmeldung eingereicht wurden und die nicht in dieser Patentschrift enthalten sind.

(30) Priorität : **11.09.87 CH 3521/87**

(43) Veröffentlichungstag der Anmeldung : **15.03.89 Patentblatt 89/11**

(45) Bekanntmachung des Hinweises auf die Patenterteilung : **12.02.92 Patentblatt 92/07**

(84) Benannte Vertragsstaaten : **BE DE FR GB IT NL**

(56) Entgegenhaltungen :
**EP-A- 0 140 298**
**DE-A- 3 333 987**
**US-A- 3 326 933**

(56) Entgegenhaltungen :
**US-A- 4 102 839**
**US-A- 4 371 651**
**CHEMICAL ABSTRACTS, Band 66, Nr. 15, 10. April 1967, Columbus, Ohio,USA; JOSE FONT et al, "Ranunsulin, protoanemonin, and anemonin. II. Chemistry of anemonin", S. 6110-6111**
**CHEMICAL ABSTRACTS, Band 107, Nr. 25, 21. Dezember 1987, Columbus, Ohio, USA; A. A. AKHREM, "Improved synthesis of methyl 5-methoxy-3-oxopentanoate", S. 727**

(73) Patentinhaber : **CIBA-GEIGY AG**
**Klybeckstrasse 141**
**CH-4002 Basel (CH)**

(72) Erfinder : **Wirth, Hermann O., Dr.**
**Lessingstrasse 24**
**W-6140 Bensheim 3 (DE)**
Erfinder : **Friedrich, Hans-Helmut**
**Am Rauhenstein 8**
**W-6147 Lautertal 2 (DE)**

**EP 0 307 358 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue etherhaltige oder thioetherhaltige 1,3-Diketone, die Verwendung von 1,3-Diketonderivaten zum Stabilisieren eines chlorhaltigen Polymerisats gegen den schädigenden Einfluss von Wärme und/oder Licht sowie die mit diesen Verbindungen stabilisierten chlorhaltigen Polymerisate.

Es ist bekannt, dass chlorhaltige Polymerisate gegen den schädigenden Einfluss von Wärme und/oder Licht, insbesondere bei der Verarbeitung zu Formteilen geschützt werden müssen. In US-A-4 102 839 wird ein Hitzestabilisator, der Metallcarboxylate und 1,3-Diketone enthält, für Polymerisate auf der Basis von Vinylchlorid beschrieben. K. Hiroi et al. beschreiben in Chem. Pharm. Bull. 27, 2338-2344 (1979) und Synthesis 621-622 (1979) die Herstellung von 1-Phenylthio-2,4-pentandion. In der DE-Offenlegungsschrift 33 33 987 wird die Verwendung von Mono-, Di- oder Triketonen, die 4-Hydroxyphenylthiogruppen enthalten, als Farbentwickler für wärmeempfindliches Aufzeichnungsmaterial beschrieben. US-A-3 326 933 offenbart etherhaltige 1,3-Diketone und deren Verwendung als Zwischenprodukte für die Herstellung von Pharmazeutika. J.M. Hoffman et al. beschreiben in J. Med. Chem. 26, 1650-1653 (1983) die Verwendung von 1-Benzyloxy-2,4-pentandion als Zwischenprodukt bei der Herstellung von einem Pyridinderivat. Aus US-A-4 371 651 ist die Verwendung von Schwefel- oder Sauerstoff-haltigen Tetraketonen als Stabilisatoren für Polyvinylchlorid bekannt. O. Dann et al. beschreiben in Archiv der Pharmazie 292, 508-518 (1959) die Herstellung von 3-Ethoxycarbonyl-7-thiaoctan-2,4-dion und J. Setsune et al. beschreiben in Chem. Express 1, 216-219 (1986) die Herstellung von 1-Phenylthio-8,10-undecandion.

Die vorliegende Erfindung betrifft Verbindungen der Formel I,

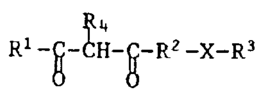

(I)

worin $R^1$ und $R^3$ unabhängig voneinander $C_1$-$C_{12}$-Alkyl, Phenyl, durch ein bis drei $C_1$-$C_{12}$-Alkylgruppen substituiertes Phenyl, $C_7$-$C_{10}$-Phenylalkyl oder am Phenylring durch ein bis drei $C_1$-$C_{12}$-Alkylgruppen substituiertes $C_7$-$C_{10}$-Phenylalkyl sind und $R^1$ zusätzlich $-R^2$-X-$R^3$ bedeutet, $R^2$ $C_2$-$C_{10}$-Alkylen darstellt, $R^4$ Wasserstoff oder $C_2$-$C_5$-Alkanoyl ist und X Sauerstoff oder Schwefel bedeutet, mit der Massgabe, dass die Verbindung der Formel

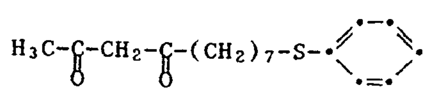

ausgeschlossen ist.

Die Verbindungen der Formel I haben beispielsweise einen günstigen Einfluss auf die Farbstabilität von chlorhaltigen Polymerisaten bei der thermoplastischen Verarbeitung. Bemerkenswert ist auch ihre langzeitstabilisierende Wirkung.

Beispiele für $R^1$ und $R^3$ als $C_1$-$C_{12}$-Alkyl sind Methyl, Ethyl, Propyl, Butyl, tert-Butyl, Pentyl, Hexyl, Heptyl, Octyl, 1,1,3,3-Tetramethylbutyl, Nonyl und Decyl. $R^1$ bedeutet bevorzugt $C_1$-$C_6$-Alkyl, insbesondere Methyl oder tert-Butyl. Eine der bevorzugten Bedeutungen von $R_3$ ist $C_4$-$C_{12}$-Alkyl.

$R^1$ und $R^3$ bedeuten als Phenyl, welches durch ein bis drei $C_1$-$C_{12}$-Alkyl-, bevorzugt $C_1$-$C_4$-Alkyl-, Gruppen substituiert sein kann, z.B. 2-Methylphenyl, 2,5-Dimethylphenyl, 2,4,6-Trimethylphenyl, 2-, 3-oder 4-n-Dodecylphenyl.

Beispiele für $R^1$ und $R^3$ als $C_7$-$C_{10}$-Phenylalkyl, welches gegebenenfalls am Phenylring durch ein bis drei $C_1$-$C_{12}$-Alkylgruppen substituiert sein kann, sind Benzyl, α-Methylbenzyl, 2-Methylbenzyl, 2,5-Dimethylbenzyl, 2,4,6-Trimethylbenzyl und 2-, 3- oder 4-n-Dodecylbenzyl. Benzyl und am Phenylring durch n-Dodecyl substituiertes Benzyl sind bevorzugt.

Beispiele für $R^2$ als $C_2$-$C_{10}$-Alkylen, welches geradkettig oder verzweigt sein kann, sind Ethylen, Trimethylen, 2-Methylethylen, Tetramethylen, 3-Methyltrimethylen, Pentamethylen, Hexamethylen, Octamethylen und Decamethylen. $R^2$ ist bevorzugt $C_2$-$C_4$-Alkylen, insbesondere $C_3$-$C_4$-Alkylen. Eine besonders bevorzugte Bedeutung von $R_2$ ist Trimethylen.

$R^4$ bedeutet als $C_2$-$C_5$-Alkanoyl z.B. Acetyl, Propanoyl, Butanoyl oder Pentanoyl. Acetyl ist bevorzugt.

Von Interesse sind Verbindungen der Formel I, mit der Massgabe das Verbindungen der Formel

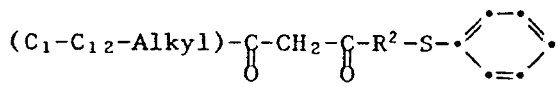 ,

worin $R^2$ wie oben definiert ist, ausgeschlossen sind.

X bedeutet bevorzugt Schwefel.

Von besonderem Interesse sind Verbindungen der Formel I, worin $R^1$ und $R^3$ unabhängig voneinander $C_1$-$C_{12}$-Alkyl, Phenyl, durch ein bis drei $C_1$-$C_{12}$-Alkylgruppen substituiertes Phenyl, Benzyl oder am Phenylring durch ein bis drei $C_1$-$C_{12}$-Alkylgruppen substituiertes Benzyl sind.

Bevorzugt sind Verbindungen der Formel I, worin $R^4$ Wasserstoff ist.

Bevorzugt sind auch Verbindungen der Formel I, worin $R^4$ $C_2$-$C_5$-Alkanoyl ist.

Ebenfalls bevorzugt sind Verbindungen der Formel I, worin $R^1$ $C_1$-$C_{12}$-Alkyl, Phenyl, durch ein bis drei $C_1$-$C_4$-Alkylgruppen substituiertes Phenyl, Benzyl oder am Phenylring durch ein bis drei $C_1$-$C_4$-Alkylgruppen substituiertes Benzyl ist.

Gemäss einer weiteren Bevorzugung bedeutet $R^1$ $C_1$-$C_6$-Alkyl, Phenyl oder durch ein bis drei Methylgruppen substituiertes Phenyl und stellt $R^3$ $C_4$-$C_{12}$-Alkyl, Phenyl, Benzyl oder am Phenylring durch n-Dodecyl substituiertes Benzyl dar.

Des weiteren sind Verbindungen der Formel I bevorzugt, worin $R^1$ $C_1$-$C_4$-Alkyl oder Phenyl bedeutet, $R^2$ $C_2$-$C_4$-Alkylen ist und $R^3$ $C_4$-$C_{12}$-Alkyl, Phenyl oder Benzyl darstellt.

Besonders bevorzugte Verbindungen der Formel I sind 1-Benzylthio-3,5-hexandion, 1-Phenylthio-4,6-heptandion, 1-(n-Dodecylthio)-4,6-heptandion und 1-Phenyloxy-4,6-heptandion.

Ein weiterer Gegenstand der Erfindung sind Zusammensetzungen enthaltend ein chlorhaltiges Polymerisat und mindestens eine Verbindung der Formel IA,

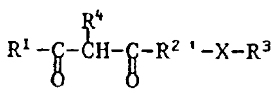 (IA)

worin $R^1$ und $R^3$ unabhängig voneinander $C_1$-$C_{12}$-Alkyl, Phenyl, durch ein bis drei $C_1$-$C_{12}$-Alkylgruppen substituiertes Phenyl, $C_7$-$C_{10}$-Phenylalkyl oder am Phenylring durch ein bis drei $C_1$-$C_{12}$-Alkylgruppen substituiertes $C_7$-$C_{10}$-Phenylalkyl sind und $R^1$ zusätzlich $-R^{2'}$-X-$R^3$ bedeutet, $R^{2'}$ $C_1$-$C_{10}$-Alkylen darstellt, $R^4$ Wasserstoff, $C_2$-$C_5$-Alkyloxycarbonyl oder $C_2$-$C_5$-Alkanoyl ist und X Sauerstoff oder Schwefel bedeutet.

$R^{2'}$ stellt bevorzugt $C_2$-$C_{10}$-Alkylen dar.

Von Interesse sind solche Zusammensetzungen, die ein chlorhaltiges Polymerisat und mindestens eine Verbindung der Formel IA enthalten, worin die Variablen $R^1$, $R^{2'}$, $R^3$, $R^4$ und X die oben unter der Formel I angegebenen, bevorzugten Bedeutungen besitzen, wobei die Variable $R^{2'}$ dem Rest $R^2$ entspricht.

$R^4$ bedeutet als $C_2$-$C_5$-Alkyloxycarbonyl z.B. Methoxycarbonyl, Ethoxycarbonyl, Propoxycarbonyl oder Butoxycarbonyl, bevorzugt tert-Butoxycarbonyl.

Beispiele für Verbindungen der Formel IA sind:

a) $H_3C-CO-CH_2-CO-CH_2CH_2-S-CH_2-$ 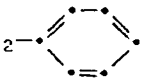

b) $H_3C-CO-CH_2-CO-CH_2CH_2-S-C_4H_9-n$

c) $H_3C-CO-CH_2-CO-CH_2-S-C_4H_9-t$

d) $H_3C-CO-CH_2-CO-CH_2-S-C_8H_{17}-t$ [1)]

e) $H_3C-CO-CH_2-CO-CH_2CH_2-S-C_{12}H_{25}-n$

f) $H_3C-CO-CH_2-CO-CH_2CH_2-S-C_{12}H_{25}-t$

g) $H_3C-CO-CH_2-CO-CH_2-S-CH_2-$ 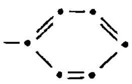

h) $H_3C-CO-CH_2-CO-CH_2CH_2CH_2-S-C_{12}H_{25}-n$

i) $t-H_9C_4-CO-CH_2-CO-CH_2CH_2-S-CH_2-$ 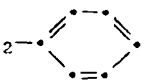

j) $t-H_9C_4-CO-CH_2-CO-CH_2CH_2-S-C_{12}H_{25}-n$

k) $t-H_9C_4-CO-CH_2-CO-CH_2CH_2-S-C_8H_{17}-n$

l) $t-H_9C_4-CO-CH_2-CO-CH_2-S-CH_2-$ 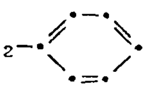

m) $t-H_9C_4-CO-CH_2-CO-CH_2-S-C_8H_{17}-t$

n) $t-H_9C_4-CO-CH_2-CO-CH_2CH_2CH_2-S-CH_2-$ 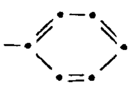

o) $t-H_9C_4-CO-CH_2-CO-CH_2CH_2\underset{CH_3}{\underset{|}{C}}H-S-CH_2-$ 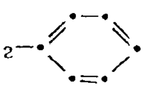

p) $t-H_9C_4-CO-CH_2-CO-CH_2CH_2\underset{CH_3}{\underset{|}{C}}H-S-C_8H_{17}-n$

---

1) $t-H_{17}C_8-$ bedeutet 1,1,3,3-Tetramethylbutyl

q) $t\text{-}H_9C_4\text{-}CO\text{-}CH_2\text{-}CO\text{-}CH_2\text{-}S\text{-}C_6H_{13}\text{-}n$

r) 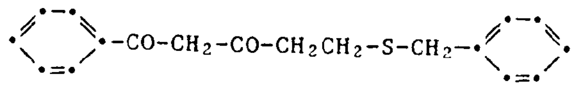

s) 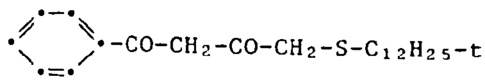

t) 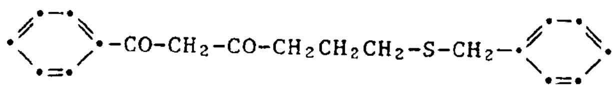

u) 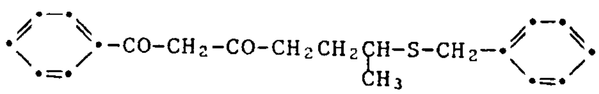

v) 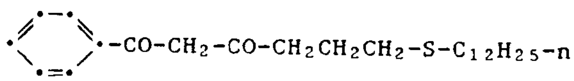

w) 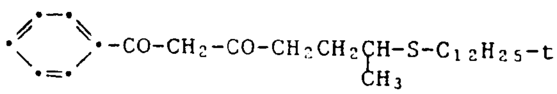 [2]

x) 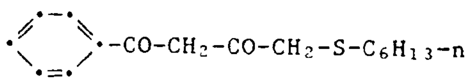

y) 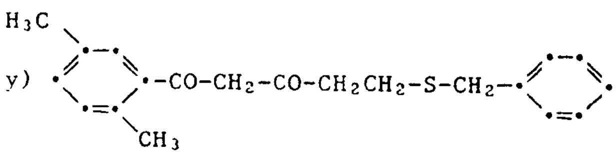

z) 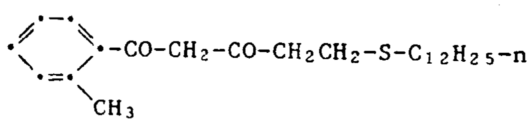

A) 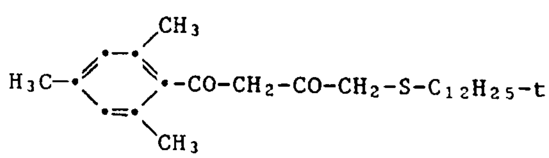

B) 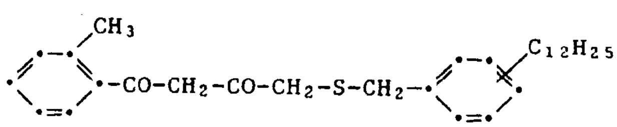

[2] $t\text{-}H_{25}C_{12}\text{-}$ bedeutet z.B. solch einen Rest, wie er für tertiäres Dodecylmercaptan in "Ullmanns Enzyklopädie der technischen Chemie, 4. Auflage, Band 23, Seiten 181 und 182, Verlag Chemie, Weinheim", beschrieben wird.

C) 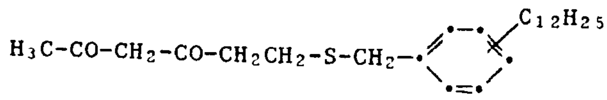

D) 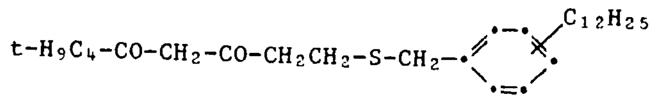

E) 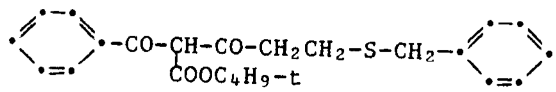

F) 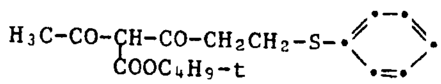

G) 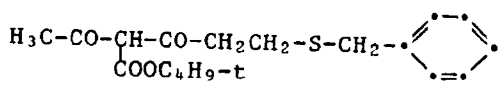

H) 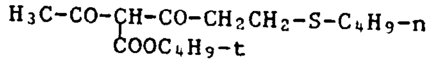

I) 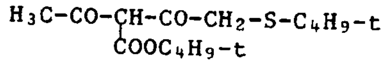

J) 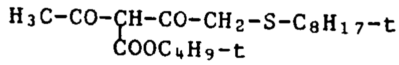

K) 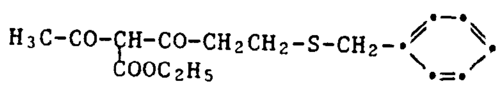

L) 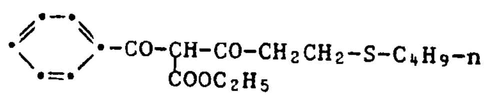

M) 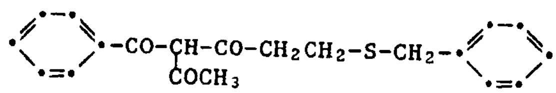

N) 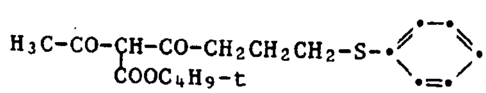

O) 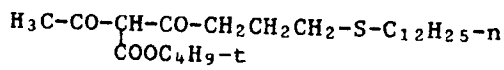

P) 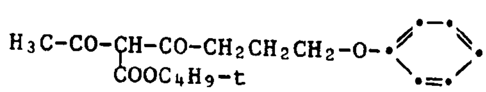

Q) 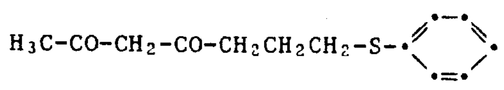

R) 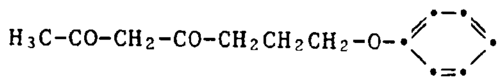

S) $t-C_4H_9-CO-CH_2-CO-CH_2CH_2CH_2-O-C_4H_9-n$

T) 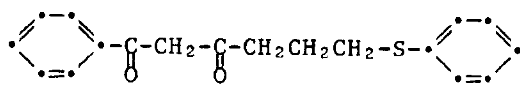

Die Verbindungen a), b), h), r), v), G), K), L), M), N), O), P), Q), R), S) und T) sind von Interesse. Bevorzugt sind die Verbindungen a), h), v), O), P), Q), R), S) und T), insbesondere die Verbindungen a), h), Q) und R).

Bei den chlorhaltigen Polymerisaten handelt es sich bevorzugt um Vinylchloridhomopolymere oder -copolymere. Als Comonomere für die Copolymerisate kommen z.B. in Frage: Vinylacetat, Vinylidenchlorid, Transdichlorethen, Ethylen, Propylen, Butylen, Maleinsäure, Acrylsäure, Fumarsäure, Itaconsäure. Weitere geeignete chlorhaltige Polymere sind nachchloriertes PVC und chlorierte Polyolefine, ferner Pfropfpolymerisate von PVC mit EVA, ABS und MBS. Bevorzugte Substrate sind auch Mischungen der vorstehend genannten Homo- und Copolymerisate, insbesondere Vinylchlorid-Homopolymerisate, mit anderen thermoplastischen oder/und elastomeren Polymeren, insbesondere mit ABS, MBS, NBR, SAN, EVA.

Weiterhin bevorzugt sind Suspensions- und Massepolymere sowie Emulsionspolymere.

Als chlorhaltiges Polymerisat sind Polyvinylchlorid sowie Copolymerisate des Vinylchlorids besonders bevorzugt.

Es ist vorteilhaft, die Verbindungen der Formel IA zusammen mit bekannten Thermostabilisatoren einzusetzen, wie z.B. Me(II)-Phenolaten, insbesondere $C_7$-$C_{20}$Alkylphenolaten, beispielsweise Nonylphenolat, oder Me(II)-Carboxylaten. Me(II) bedeutet z.B. Ba, Ca, Mg, Cd oder Zn. Bei den Carboxylaten handelt es sich bevorzugt um Salze von Carbonsäuren mit 7 bis 20 C-Atomen, beispielsweise Benzoate, Alkenoate oder Alkanoate, bevorzugt Stearate, Oleate, Laurate, Palmitate, Hydroxystearate oder 2-Ethylhexanoate. Besonders bevorzugt sind Stearate, Oleate und p-tert-Butylbenzoate.

Zusätzlich können die chlorhaltigen Polymerisate in üblichen Mengen herkömmliche PVC-Stabilisatoren enthalten, wie beispielsweise Epoxiverbindungen, wie epoxidierte Oele und vorzugsweise epoxidierte Fettsäureester, insbesondere epoxidiertes Sojabohnenöl, sowie Phosphite, bevorzugt solche der Formeln

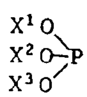 und 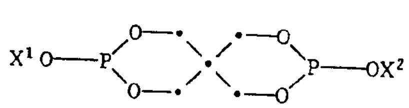 ,

worin $X^1$, $X^2$ und $X^3$ unabhängig voneinander $C_4$-$C_{18}$-Alkyl, Phenyl oder durch ein bis drei $C_1$-$C_{12}$-Alkylgruppen substituiertes Phenyl bedeuten.

Besonders bevorzugte organische Phosphite sind Distearyl-pentaerythritdiphosphit, Trisnonylphenylphosphit und Phenyl-didecyl-phosphit.

Die bekannten Thermostabilisatoren (z.B. Carboxylate) können in dem zu stabilisierenden Material in einer dem Fachmann bekannten Konzentration vorliegen, wie zum Beispiel in Mengen von 0,05 bis 5 Gew.%.

Die Phosphite werden z.B. in Konzentration von 0,3 bis 5, vorzugsweise 0,5 bis 1 Gew.% und die Epoxiverbindungen, wie z.B. das epoxidierte Sojabohnenöl, in Konzentrationen von 2 bis 8, vorzugsweise 1 bis 3 Gew.% eingesetzt.

Die Verbindungen der Formel IA werden beispielsweise in Mengen von 0,05 bis 1, bevorzugt 0,1 bis 0,5 Gew.% in das chlorhaltige Polymerisat eingearbeitet.

Die Angabe Gew.% bezieht sich jeweils auf das zu stabilisierende Material.

Bevorzugt sind chlorhaltige Polymerisate enthaltend mindestens eine Verbindung der Formel IA und zusätzlich eine Epoxiverbindung und mindestens ein Me(II)-Carboxylat und/oder Me(II)-Phenolat und gegebenenfalls ein Phosphit; Me(II) bedeutet Ca, Ba, Mg, Cd oder Zn.

Gemäss einer weiteren Bevorzugung enthalten die erfindungsgemässen chlorhaltigen Polymerisate mindestens eine Verbindung der Formel IA, epoxidiertes Sojabohnenöl und mindestens ein Me(II)-Carboxylat, wobei Me(II) Ca, Ba, Mg oder Zn, insbesondere Zn, bedeutet. Gemische aus Ba/Zn- oder Ca/Zn-Carboxylaten

werden dabei als Costabilisatoren besonders bevorzugt.

Je nach dem Verwendungszweck der Polymerisate können vor oder bei der Einarbeitung der Stabilisatoren auch weitere Zusätze eingearbeitet werden, wie zum Beispiel phenolische Antioxidantien, Gleitmittel (bevorzugt Montanwachse oder Glycerinester), Fettsäureester, Paraffine, Weichmacher, Füllstoffe, Russ, Asbest, Kaolin, Talk, Glasfasern, Modifikatoren (wie etwa Schlapzäh-Zusätze), optische Aufheller, Pigmente, Lichtschutzmittel, UV-Absorber, Flammschutzmittel oder Antistatika.

Die Einarbeitung der Stabilisatorkomponenten in das chlorhaltige Polymerisat erfolgt am günstigsten, wie üblich, auf einem 2-Walzenstuhl bei Temperaturen zwischen 150 und 200°C. Im allgemeinen lässt sich eine genügende Homogenisierung innerhalb von 5 bis 15 Minuten erreichen. Die Zugabe der Komponenten kann einzeln oder gemeinsam als Vorgemisch erfolgen. Als zweckmässig hat sich ein flüssiges Vorgemisch erwiesen, d.h. es wird in Gegenwart von indifferenten Lösungsmitteln und/oder Weichmachern gearbeitet.

Die Verbindungen der Formel IA, worin $R^4$ Wasserstoff bedeutet, können in Analogie zu bekannten Verfahren hergestellt werden, z.B. durch Umsetzung einer Verbindung der Formel II,

$$R^1-\underset{\underset{O}{\|}}{C}-CH_3 \qquad (II)$$

worin $R^1$ die oben angegebene Bedeutung hat, mit einer Verbindung der Formel III,

$$R'-O-\underset{\underset{O}{\|}}{C}-R^{2'}-X-R^3 \qquad (III)$$

worin X, $R^{2'}$ und $R^3$ die oben angegebene Bedeutung besitzen und R′ bevorzugt $C_1$-$C_3$-Alkyl ist, in Gegenwart einer stark basischen Verbindung, wie z.B. einem Alkali- oder Magnesium-alkoholat, -hydrid oder -amid, insbesondere Natriummethylat oder Natriummethylat. Die Umsetzung findet vorzugsweise in einem inerten Lösungsmittel, wie z.B. Toluol, Xylol oder Tetrahydrofuran statt. Die Reaktionstemperatur liegt zweckmässig bei "20°C bis Siedetemperatur des gewählten Lösungsmittels". Das primäre Reaktionsprodukt ist das Alkalimetall-oder Magnesiumchelat des entsprechenden Diketoderivats, das durch Säuren, wie z.B. Salzsäure, Schwefelsäure oder Ameisensäure, gespalten werden kann. Man erhält als Reaktionsprodukt die Verbindung der Formel IA mit $R^4$ = H, die, falls gewünscht, auch unmittelbar als Stabilisator eingesetzt werden kann. Das Rohprodukt kann nach üblichen Methoden (z.B. Umkristallisation oder Destillation) aufgearbeitet werden.

Die Verbindungen der Formel III können ebenfalls in Analogie zu bekannten Verfahren hergestellt werden, beispielsweise durch Umsetzung einer Verbindung der Formel IV

$$R'-O-\underset{\underset{O}{\|}}{C}-R^{2'}-XH \qquad (IV)$$

mit einer Verbindung der Formel V,

$$Y-R^3 \qquad (V)$$

worin X, R′, $R^{2'}$ und $R^3$ die oben angegebenen Bedeutungen besitzen und Y Halogen, bevorzugt Cl, darstellt. Die Reaktionstemperatur liegt vorzugsweise bei 20-60°C.

Bedeutet $R^{2'}$ in den Verbindungen der Formel III Dimethylen oder 1,2-Propylen, so können diese Verbindungen auch durch Umsetzung von

$$R'-O-\underset{\underset{O}{\|}}{C}-CH=CHR'' \qquad (VI)$$

mit

$$H-X-R^3 \qquad (VII)$$

hergestellt werden, wobei X, R′ und $R^3$ wie oben definiert sind und R″ Wasserstoff oder Methyl bedeutet.

Die Verbindungen der Formel IA, worin $R^4$ $C_2$-$C_5$-Alkyloxycarbonyl oder $C_2$-$C_5$-Alkanoyl ist, können gemäss dem folgenden Reaktionsschema hergestellt werden:

A)

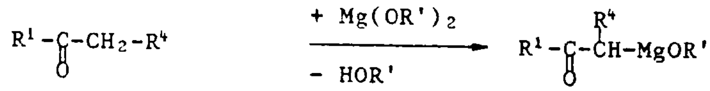


(VIII) (IX)

B)

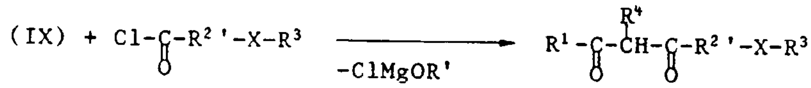


(X) (XI)

Die Variablen $R^1$, $R^{2'}$, $R^3$, $R^4$, X und R′ besitzen die oben angegebenen Bedeutungen.

Die Umsetzungen A und B können nacheinander in einem Reaktionsgefäss ohne Isolierung der Verbindung IX durchgeführt werden. Das Reaktionsmedium ist zweckmässig ein inertes Lösungsmittel, wie z.B. Toluol oder ein Ether, insbesondere Diethylether oder Tetrahydrofuran. Die Umsetzung A findet bevorzugt bei 5 bis 15°C statt und die Umsetzung B bei 0 bis 25°C.

Bedeutet $R^{2'}$ Trimethylen, können wie Verbindungen der Formel X z.B. durch Umsetzung einer Verbindung der Formel VII mit γ-Butyrolacton und anschliessender Ueberführung der erhaltenen Carbonsäure in das entsprechende Carbonsäurechlorid hergestellt werden.

Die Verbindungen der Formel IA, worin $R^4$ Wasserstoff bedeutet, können auch durch Decarboxylierung der Verbindung XIa hergestellt werden.

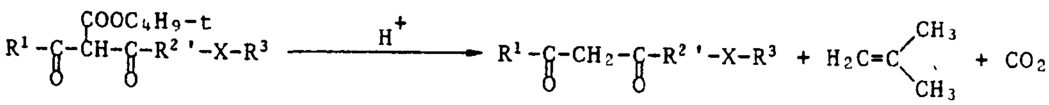


(XIa)

Die Decarboxylierung findet zweckmässig unter kationischen Bedingungen bei ca. 60 bis 80°C statt.

Einschlägige Untersuchungen zu der oben beschriebenen Synthesemethode gehen auf A. Treibs und K. Hintermeier; Chem. Ber., 1163-1166 (1954) zurück.

Ferner ist es möglich, Verbindungen der Formel IA, worin $R^4$ Wasserstoff ist, über das Triacetylmethanderivat der Formel XIb

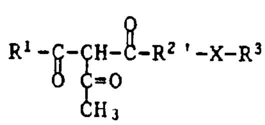

(XIb)

als Zwischenstufe herzustellen. Durch Abspaltung eines Acetylrestes geht die Verbindung der Formel XIb in die entsprechende Verbindung der Formel IA über.

Die Verbindungen der Formel XIb können auch durch Umsetzung einer Verbindung der Formel VIIIa

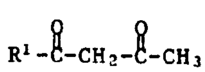

(VIIIa)

mit einem Säurechlorid der Formel X hergestellt werden.

Die für die Synthese der erfindungsgemässen Verbindungen erforderlichen Ether- oder Thioether-carbonsäurechloride der Formel X können nach konventionellen Verfahren hergestellt werden, wie z.B. durch Umsetzung der Ether- oder Thioether-carbonsäuren mit Thionylchlorid.

Die Verbindungen der Formel IA, worin $R^{2'}$ 1,2-Propylen ist und $R^4$ Wasserstoff bedeutet, können z.B. auch nach folgendem Reaktionsschema in an sich bekannter Weise hergestellt werden:

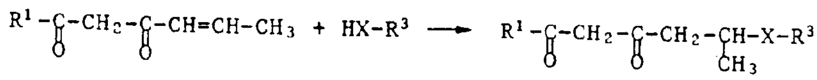

(XII)

Die in den oben beschriebenen Herstellungsverfahren verwendeten Ausgangsprodukte können, sofern sie nicht im Handel erhältlich oder vorbeschrieben sind, nach bekannten Verfahren hergestellt werden.

Die folgenden Beispiele erläutern die Erfindung weiter. Alle Mengenangaben entsprechen Gewichtsteilen, soweit nicht anders angegeben.

Beispiel 1: Herstellung von 1-Benzylthio-4-tert-butoxycarbonyl-3,5-hexandion

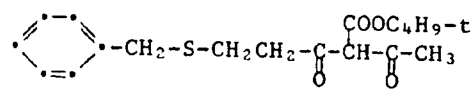

In einem 1 ℓ Kolben mit Rührer, Thermometer, Tropftrichter und Rückflusskühler werden unter Feuchtigkeitsausschluss 10,7 g (0,44 Mol) Magnesium, 2 ml Chloroform und 46 g (1 Mol) absolutes Ethanol vorgelegt und 1 Stunde am Rückfluss unter Rühren erwärmt. Anschliessend werden 300 ml Diethylether zugetropft und das Gemisch 1,5 Stunden gerührt. Dann wird das Gemisch auf ca. 12°C abgekühlt. Bei dieser Temperatur werden 63,2 g (0,4 Mol) Acetessigsäure-tert-butylester zugetropft. Das Reaktionsgemisch wird weiter auf ca. -3°C abgekühlt und bei dieser Temperatur werden innerhalb von 45 Minuten 85,8 g (0,4 Mol) Benzylthiomethylessigsäurechlorid in 100 ml absolutem Diethylether zugetropft. Anschliessend wird das Gemisch noch 1 Stunde bei ca. -3°C gerührt und dann weitere 2 Stunden ohne Kühlung, wobei Raumtemperatur erreicht wird. Das Reaktionsgemisch wird in 400 g Eiswasser und 48 g konz. $H_2SO_4$ eingerührt. Die organische Phase wird abgetrennt und mit 150 ml Wasser, 100 ml Bicarbonat und wiederum 150 ml Wasser gewaschen, anschliessend getrocknet und eingeengt.

Ausbeute: 128,6 g (= 95,5 % der Theorie)

Brechungsindex: $n^{20}_D$= 1,5284

Beispiele 2-9

Die Herstellung der folgenden Verbindungen erfolgt in Analogie zu Beispiel 1.

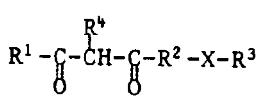

Tabelle 1:

| Bsp. | $R^1$ | $R^2$ | $R^3$ | $R^4$ | X | Brechungs-index |
|---|---|---|---|---|---|---|
| 2 | $-CH_3$ | $-CH_2CH_2-$ | $-C_4H_9$-n | $-COOC_4H_9$-t | S | $n_D^{20}$ = 1,4813 |
| 3 | $-CH_3$ | $-CH_2-$ | $-C_4H_9$-t | $-COOC_4H_9$-t | S | $n_D^{20}$ = 1,4913 |
| 4 | $-CH_3$ | $-CH_2-$ | $-C_8H_{17}$-t | $-COOC_4H_9$-t | S | $n_D^{20}$ = 1,4930 |
| 5 | $-CH_3$ | $-CH_2CH_2-$ | 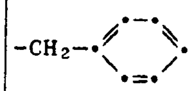 | $-COOC_2H_5$ | S | $n_D^{20}$ = 1,5486 |
| 6 | 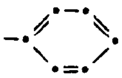 | $-CH_2CH_2-$ | $-C_4H_9$-n | $-COOC_2H_5$ | S | $n_D^{20}$ = 1,5376 |
| 7 | $-CH_3$ | $-CH_2CH_2CH_2-$ | 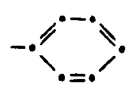 | $-COOC_4H_9$-t | S | $n_D^{20}$ = 1,5334 |
| 8 | $-CH_3$ | $-CH_2CH_2CH_2-$ | $-C_{12}H_{25}$-n | $-COOC_4H_9$-t | S | $n_D^{20}$ = 1,4768 |
| 9 | $-CH_3$ | $-CH_2CH_2CH_2-$ | 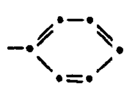 | $-COOC_4H_9$-t | O | $n_D^{20}$ = 1,5106 |

Beispiel 10: Herstellung von 1-Benzylthio-3,5-hexandion

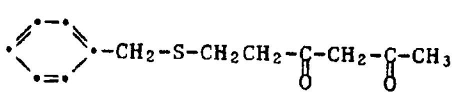

In einem 250 ml Kolben mit Rührer, Thermometer, Rückflusskühler und Blasenzähler werden 101 g (0,3 Mol) 1-Benzylthio-4-tert-butoxycarbonyl-3,5-hexandion (s. Beispiel 1) und 1,0 g p-Toluolsulfonsäure 225 Minuten auf 70-75°C erwärmt, bis die Gasentwicklung ($CO_2$) beendet ist. Es werden 100 ml Diethylether zugegeben und das Gemisch zuerst mit 50 ml Bicarbonat und dann mit 100 ml Wasser gewaschen, anschliessend eingeengt und fraktioniert.

Ausbeute: 48,0 g (= 67,7 % der Theorie)

Siedepunkt: 122°C bei 0,001 bar

Beispiele 11-16:

Die Herstellung der folgenden Verbindungen erfolgt in Analogie zu Beispiel 10 unter Verwendung der entsprechenden Ausgangsprodukte gemäss Tabelle 1.

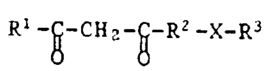

Tabelle 2:

| Bsp. | $R^1$ | $R^2$ | $R^3$ | X | Brechungsindex Schmelzpunkt |
|---|---|---|---|---|---|
| 11 | $-CH_3$ | $-CH_2CH_2-$ | $-C_4H_9$-n | S | $n_D^{20}$ = 1,4946 |
| 12 | $-CH_3$ | $-CH_2-$ | $-C_4H_9$-t | S | $n_D^{20}$ = 1,5005 |
| 13 | $-CH_3$ | $-CH_2-$ | $-C_8H_{17}$-t | S | $n_D^{20}$ = 1,5027 |
| 14 | $-CH_3$ | $-CH_2CH_2CH_2-$ | 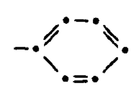 | S | Smp. = 29°C |
| 15 | $-CH_3$ | $-CH_2CH_2CH_2-$ | $-C_{12}H_{25}$-n | S | Smp. = 33°C |
| 16 | $-CH_3$ | $-CH_2CH_2CH_2-$ | 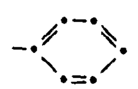 | O | Smp. = 39°C |

Beispiel 17: Herstellung von 1-Benzylthio-4-benzoyl-3,5-hexandion

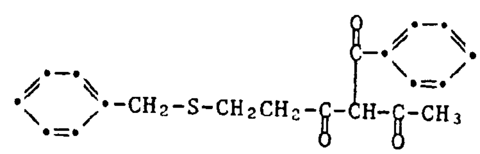

Die Herstellung erfolgt in Analogie zu Beispiel 1, wobei anstelle von 0,4 Mol Acetessigsäure-tert-butylester 0,4 Mol Benzoylaceton eingesetzt werden.
Ausbeute: 129,8 g (= 95,3 % der Theorie)
Brechungsindex: $n^{20}_D$ = 1,6000

Beispiel 18: Herstellung von 1-Benzylthio-5-phenyl-3,5-pentandion

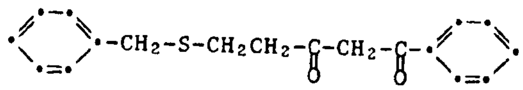

In einem 100 ml Kolben mit Thermometer, Rührer, Tropfrichter und Rückflusskühler werden 34,0 g (0,1 Mol) 1-Benzylthio-4-benzoyl-3,5-hexandion (s. Beispiel 17) vorgelegt. Unter Rühren werden innerhalb von 40 Minuten 19,8 g (0,11 Mol) Natriummethylatlösung (30 % in Methanol) zugetropft. Die Reaktionstemperatur steigt dabei von Raumtemperatur auf ca. 32°C an. Das Gemisch wird noch 1 Stunde bei Raumtemperatur und weitere 3 Stunden bei ca. 45°C gerührt, anschliessend auf Raumtemperatur abgekühlt und in 200 ml Wasser und 12 g konz. $H_2SO_4$ eingerührt. Es werden 180 ml Diethylether zugegeben und die organische Phase wird abgetrennt. Die wässsrige Phase wird nochmals mit 50 ml Diethylether gewaschen. Die vereinigten organischen Phasen werden mit Wasser, Natriumbicarbonat und wiederum mit Wasser gewaschen, anschliessend getrocknet, eingeengt und fraktioniert. Das erhaltene Produkt mit einem Siedepunkt von 180-183°C bei 0,001 bar wird aus 20 ml Isopropanol umkristallisiert.
Schmelzpunkt: 55-56°C

Beispiel 19: Herstellung von 1-n-Hexylthio-5,5-dimethyl-2,4-hexandion

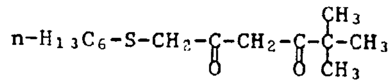

In einem Kolben mit Thermometer, Rührer, Tropftrichter und Destillationsbrücke mit Vorlage werden 250 ml Toluol und 99 g (0,55 Mol) Natriummethylatlösung (30 % in Methanol) vorgelegt und 108,4 g eines Methanol/Toluol-Gemisches bis zu einer Reaktionstemperatur von 109°C abdestilliert. Bei gleicher Temperatur werden dann 60 g (0,6 Mol) Pinakolin und innerhalb 20 Minuten 95 g (0.5 Mol) n-Hexylthioessigsäuremethylester zugetropft. Hierbei werden nochmals 32,5 g eines Methanol/Toluol Gemisches abdestilliert. Das Gemisch wird dann noch 35 Minuten bei 106°C nachgerührt und in Analogie zu Beispiel 18 aufgearbeitet. Der Rückstand wird eingeengt und anschliessend im Vakuum fraktioniert.
Siedepunkt: 130-140°C bei 0,2 bar
Brechungsindex: $n^{20}_D$ = 1,4890

Beispiel 20: Herstellung von 1-n-Hexylthio-4-phenyl-2,4-butandion

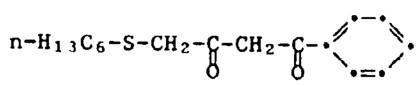

Die Verbindung wird in Analogie zu Beispiel 19 hergestellt.
Siedepunkt: 144-148°C bei 0,1 bar
Brechungsindex: $n^{20}_D$ = 1,5786

Beispiel 21: Herstellung von 1-n-Butoxy-7,7-dimethyl-4,6-octandion

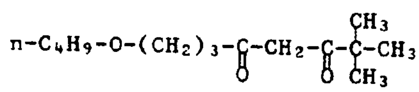

Die Verbindung wird in Analogie zu Beispiel 19 hergestellt.
Siedepunkt: 112-116°C bei 1 bar
Brechungsindex: $n^{20}_D$ = 1,4532

Beispiel 22:

Eine Trockenmischung bestehend aus 100 Teilen PVC (K-Wert 64), 3 Teilen epoxidiertem Sojabohnenöl, 0,15 Teilen Zn-Stearat, 0,15 Teilen Ca-Stearat und 0,3 Teilen des in Tabelle 3 angegebenen Stabilisators wird auf einem Mischwalzwerk 5 Minuten bei 180°C gewalzt. Vom gebildeten 0,3 mm dicken Walzfell werden Folienmuster in einem Testofen (®Mathis Thermotester Typ LTF-ST) bei 190°C thermisch belastet und im angegebenen Zeitintervall an einem Prüfmuster der "Yellowness Index" (YI) nach ASTM D 1925 bestimmt. Die Ergebnisse sind in Tabelle 3 zusammengefasst.

Tabelle 3:

| Stabilisator | YI nach Belastungszeit in Minuten 3 | 5 | 7 | 9 | 11 | 13 | 15 |
|---|---|---|---|---|---|---|---|
| ohne | 42,3 | 47,1 | 48,4 | 52,4 | 67,4 | 83,8 | 95,1 |
| Verbindung aus Beispiel 10 | 4,9 | 6,3 | 8,9 | 15,4 | 37,7 | 58,6 | 84,4 |

Beispiel 23:

Eine Trockenmischung bestehend aus 100 Teilen S-PVC (K-Wert 70), 17 Teilen Dioctylphthalat, 3 Teilen epoxidiertem Sojabohnenöl, 0,33 Teilen Zn-Oleat, 0,53 Teilen Ba-p-t-Butylbenzoat, 0,70 Teilen Phenyl-diisodecylphosphit, 0,44 Teilen ®SHELL SOL A (aromatisches Kohlenwasserstoffgemisch) und 0,2 Teilen des in Tabelle 4 angegebenen Stabilisators wird auf einem Mischwalzwerk 5 Minuten bei 180°C gewalzt. Vom gebildeten 0,3 mm dicken Walzfell werden Folienmuster in einem Testofen (®Mathis Thermotester Typ LTF-St) bei 180°C thermisch belastet und im angegebenen Zeitintervall an einem Prüfmuster der "Yellowness Index" (YI) nach ASTM D 1925 bestimmt. Die Ergebnisse sind in Tabelle 4 zusammengefasst.

Tabelle 4:

| Stabilisator | YI nach Belastungszeit in Minuten 0 | 5 | 10 | 15 | 20 | 25 | 30 |
|---|---|---|---|---|---|---|---|
| ohne | 9,5 | 12,0 | 15,1 | 19,5 | 26,0 | 28,8 | 29,3 |
| Verbindung aus Beispiel 8 | 0,9 | 1,4 | 1,9 | 2,0 | 2,6 | 3,3 | 4,4 |
| Verbindung aus Beispiel 9 | 0,9 | 1,2 | 1,6 | 2,0 | 2,4 | 3,4 | 4,3 |
| Verbindung aus Beispiel 14 | 0,7 | 0,7 | 1,3 | 1,1 | 1,4 | 1,7 | 2,3 |
| Verbindung aus Beispiel 15 | 0,8 | 0,7 | 1,2 | 1,4 | 1,7 | 2,0 | 3,0 |
| Verbindung aus Beispiel 16 | 1,1 | 0,9 | 1,2 | 1,5 | 1,4 | 1,9 | 2,5 |

## Patentansprüche

1. Verbindungen der Formel I,

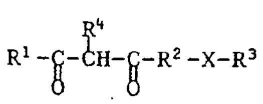

(I)

worin $R^1$ und $R^3$ unabhängig voneinander $C_1$-$C_{12}$-Alkyl, Phenyl, durch ein bis drei $C_1$-$C_{12}$-Alkylgruppen substituiertes Phenyl, $C_7$-$C_{10}$-Phenylalkyl oder am Phenylring durch ein bis drei $C_1$-$C_{12}$-Alkylgruppen substituiertes $C_7$-$C_{10}$-Phenylalkyl sind und $R^1$ zusätzlich -$R^2$-X-$R^3$ bedeutet, $R^2$ $C_2$-$C_{10}$-Alkylen darstellt, $R^4$ Wasserstoff oder $C_2$-$C_5$-Alkanoyl ist und X Sauerstoff oder Schwefel bedeutet, mit der Massgabe, dass die Verbindung der Formel

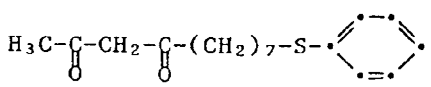

ausgeschlossen ist.

2. Verbindungen gemäss Anspruch 1, worin X Schwefel bedeutet.

3. Verbindungen gemäss Anspruch 1, worin $R^1$ und $R^3$ unabhängig voneinander $C_1$-$C_{12}$-Alkyl, Phenyl, durch ein bis drei $C_1$-$C_{12}$-Alkylgruppen substituiertes Phenyl, Benzyl oder am Phenylring durch ein bis drei $C_1$-$C_{12}$-Alkylgruppen substituiertes Benzyl sind.

4. Verbindungen gemäss Anspruch 1, worin $R^4$ Wasserstoff ist.

5. Verbindungen gemäss Anspruch 1, worin $R^4$ $C_2$-$C_5$-Alkanoyl ist.

6. Verbindungen gemäss Anspruch 1, worin $R^1$ $C_1$-$C_{12}$-Alkyl, Phenyl, durch ein bis drei $C_1$-$C_4$-Alkylgruppen substituiertes Phenyl, Benzyl oder am Phenylring durch ein bis drei $C_1$-$C_4$-Alkylgruppen substituiertes Benzyl ist.

7. Verbindungen gemäss Anspruch 1, worin $R^1$ $C_1$-$C_6$-Alkyl, Phenyl oder durch ein bis drei Methylgruppen substituiertes Phenyl ist und $R^3$ $C_4$-$C_{12}$-Alkyl, Phenyl, Benzyl oder am Phenylring durch n-Dodecyl substituiertes Benzyl darstellt.

8. Verbindungen gemäss Anspruch 1, worin $R^2$ $C_2$-$C_4$-Alkylen ist.

9. Verbindungen gemäss Anspruch 1, worin $R^1$ $C_1$-$C_4$-Alkyl oder Phenyl bedeutet, $R^2$ $C_2$-$C_4$-Alkylen ist und $R^3$ $C_4$-$C_{12}$-Alkyl, Phenyl oder Benzyl darstellt.

10. Die Verbindungen 1-Benzylthio-3,5-hexandion, 1-Phenylthio-4,6-heptandion, 1-(n-Dodecylthio)-4,6-heptandion und 1-Phenyloxy-4,6-heptandion gemäss Anspruch 1.

11. Zusammensetzung enthaltend ein chlorhaltiges Polymerisat und mindestens eine Verbindung der Formel IA,

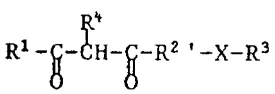

(IA)

worin $R^1$ und $R^3$ unabhängig voneinander $C_1$-$C_{12}$-Alkyl, Phenyl, durch ein bis drei $C_1$-$C_{12}$-Alkylgruppen substituiertes Phenyl, $C_7$-$C_{10}$-Phenylalkyl oder am Phenylring durch ein bis drei $C_1$-$C_{12}$-Alkylgruppen substituiertes $C_7$-$C_{10}$-Phenylalkyl sind und $R^1$ zusätzlich -$R^{2'}$-X-$R^3$ bedeutet, $R^{2'}$ $C_1$-$C_{10}$-Alkylen darstellt, $R^4$ Wasserstoff, $C_2$-$C_5$-Alkyloxycarbonyl oder $C_2$-$C_5$-Alkanoyl ist und X Sauerstoff oder Schwefel bedeutet.

12. Zusammensetzung gemäss Anspruch 11, worin X Schwefel bedeutet.

13. Zusammensetzung gemäss Anspruch 11, worin $R^{2'}$ $C_2$-$C_{10}$-Alkylen ist.

14. Zusammensetzung gemäss Anspruch 11, enthaltend zusätzlich eine Epoxiverbindung und mindestens ein Me(II)-Carboxylat und/oder Me(II)-Phenolat, wobei Me(II) Ba, Ca, Mg, Cd oder Zn bedeutet.

15. Zusammensetzung gemäss Anspruch 11, enthaltend zusätzlich epoxidiertes Sojabohnenöl und mindestens ein Me(II)-Carboxylat, wobei ME(II) Ba, Ca, Mg oder Zn bedeutet.

16. Zusammensetzung gemäss Anspruch 14, enthaltend zusätzlich ein Phosphit.

17. Zusammensetzung gemäss Anspruch 11, dadurch gekennzeichnet, dass das chlorhaltige Polymerisat Polyvinylchlorid ist.

18. Verwendung von Verbindungen der Formel IA gemäss Anspruch 11 zum Stabilisieren von chlorhaltigen Polymerisaten gegen den schädigenden Einfluss von Wärme und/oder Licht.

## Claims

1. A compound of the formula I

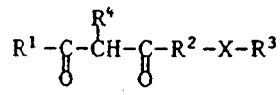

(I)

in which $R^1$ and $R^3$ independently of one another are $C_1$-$C_{12}$alkyl, phenyl, phenyl which is substituted by one to three $C_1$-$C_{12}$alkyl groups, $C_7$-$C_{10}$phenylalkyl or $C_7$-$C_{10}$phenylalkyl which is substituted on the phenyl ring by one to three $C_1$-$C_{12}$alkyl groups and $R^1$ is additionally -$R^2$-X-$R^3$, $R^2$ is $C_2$-$C_{10}$alkylene, $R^4$ is hydrogen or $C_2$-$C_5$alkanoyl and X is oxygen or sulfur, subject to the proviso that the compound of the formula

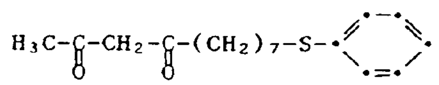

is excluded.

2. A compound according to claim 1, in which X is sulfur.

3. A compound according to claim 1, in which $R^1$ and $R^3$ independently of one another are $C_1$-$C_{12}$alkyl, phenyl, phenyl which is substituted by one to three $C_1$-$C_{12}$alkyl groups, benzyl or benzyl which is substituted on the phenyl ring by one to three $C_1$-$C_{12}$alkyl groups.

4. A compound according to claim 1, in which $R^4$ is hydrogen.

5. A compound according to claim 1, in which $R^4$ is $C_2$-$C_5$alkanoyl.

6. A compound according to claim 1, in which $R^1$ is $C_1$-$C_{12}$alkyl, phenyl, phenyl which is substituted by one to three $C_1$-$C_4$alkyl groups, benzyl or benzyl which is substituted on the phenyl ring by one to three $C_1$-$C_4$alkyl groups.

7. A compound according to claim 1, in which $R^1$ is $C_1$-$C_6$alkyl, phenyl or phenyl which is substituted by one to three methyl groups and $R^3$ is $C_4$-$C_{12}$alkyl, phenyl, benzyl or benzyl which is substituted on the phenyl ring by n-dodecyl.

8. A compound according to claim 1, in which $R^2$ is $C_2$-$C_4$alkylene.

9. A compound according to claim 1, in which $R^1$ is $C_1$-$C_4$alkyl or phenyl, $R^2$ is $C_2$-$C_4$alkylene and $R^3$ is $C_4$-$C_{12}$alkyl, phenyl or benzyl.

10. The compounds 1-benzylthio-3,5-hexanedione, 1-phenylthio-4,6-heptanedione, 1-(n-dodecylthio)-4,6-heptanedione and 1-phenyloxy-4,6-heptanedione according to claim 1.

11. A composition containing a chlorine-containing polymer and at least one compound of the formula IA

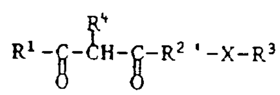

(IA)

in which $R^1$ and $R^3$ independently of one another are $C_1$-$C_{12}$alkyl, phenyl, phenyl which is substituted by one to three $C_1$-$C_{12}$alkyl groups, $C_7$-$C_{10}$phenylalkyl or $C_7$-$C_{10}$phenylalkyl which is substituted on the phenyl ring by one to three $C_1$-$C_{12}$alkyl groups and $R^1$ is additionally -$R^{2'}$-X-$R^3$, $R^{2'}$ is $C_1$-$C_{10}$alkylene, $R^4$ is hydrogen, $C_2$-$C_5$alkoxycarbonyl or $C_2$-$C_5$alkanoyl and X is oxygen or sulfur.

12. A composition according to claim 11, in which X is sulfur.

13. A composition according to claim 11, in which $R^{2'}$ is $C_2$-$C_{10}$alkylene.

14. A composition according to claim 11, containing, in addition, an epoxy compound and at least one Me(II) carboxylate and/or Me(II) phenate in which Me(II) is Ba, Ca, Mg, Cd or Zn.

15. A composition according to claim 11, containing, in addition, epoxidized soya bean oil and at least one Me(II) carboxylate in which Me(II) is Ba, Ca, Mg or Zn.

16. A composition according to claim 14, containing, in addition, a phosphite.

17. A composition according to claim 11, wherein the chlorine-containing polymer is polyvinyl chloride.

18. The use of a compound of the formula IA according to claim 11 for stabilizing chlorine-containing polymers against the harmful effects of heat and/or light.

## Revendications

1. Composés répondant à la formule I :

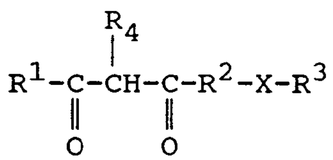

(I)

dans laquelle $R^1$ et $R^3$ représentent chacun, indépendamment l'un de l'autre, un alkyle en $C_1$-$C_{12}$, un phényle, un phényle porteur d'un à trois alkyles en $C_1$-$C_{12}$, un phényl-alkyle en $C_7$-$C_{10}$ ou un phényl-alkyle en $C_7$-$C_{10}$ dont le phényle porte d'un à trois alkyles en $C_1$-$C_{12}$, $R^1$ pouvant en outre représenter un radical -$R^2$-X-$R^3$, $R^2$ représente un alkylène en $C_2$-$C_{10}$, $R^4$ représente l'hydrogène ou un alcanoyle en $C_2$-$C_5$ et X représente l'oxygène ou le soufre, avec la condition que le composé de formule :

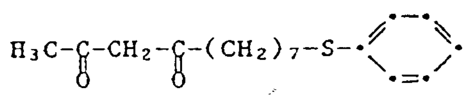

soit exclu.

2. Composés selon la revendication 1 dans lesquels X représente le soufre.

3. Composés selon la revendication 1 dans lesquels $R^1$ et $R^3$ représentent chacun, indépendamment l'un de l'autre, un alkyle en $C_1$-$C_{12}$, un phényle, un phényle porteur d'un à trois alkyles en $C_1$-$C_{12}$, un benzyle ou un benzyle dont le cycle phényle porte d'un à trois alkyles en $C_1$-$C_{12}$.

4. Composés selon la revendication 1 dans lesquels $R^4$ représente l'hydrogène.

5. Composés selon la revendication 1 dans lesquels $R^4$ représente un alcanoyle en $C_2$-$C_5$.

6. Composés selon la revendication 1 dans lesquels $R^1$ représente un alkyle en $C_1$-$C_{12}$, un phényle, un phényle porteur d'un à trois alkyles en $C_1$-$C_4$, un benzyle ou un benzyle dont le phényle porte d'un à trois alkyles en $C_1$-$C_4$.

7. Composés selon la revendication 1 dans lesquels $R^1$ représente un alkyle en $C_1$-$C_6$, un phényle ou un phényle porteur d'un à trois radicaux méthyles et $R^3$ représente un alkyle en $C_4$-$C_{12}$, un phényle, un benzyle ou un benzyle dont le phényle porte un radical n-dodécyle.

8. Composés selon la revendication 1 dans lesquels $R^2$ représente un alkylène en $C_2$-$C_4$.

9. Composés selon la revendication 1 dans lesquels $R^1$ représente un alkyle en $C_1$-$C_4$ ou un phényle, $R^2$ représente un alkylène en $C_2$-$C_4$ et $R^3$ représente un alkyle en $C_4$-$C_{12}$, un phényle ou un benzyle.

10. Composés selon la revendication 1, en l'espèce la benzylthio-1 hexane-dione-3,5, la phénylthio-1 heptane-dione-4,6, la (n-dodécylthio)-1 heptane-dione-4,6 et la phénoxy-1 heptane-dione-4,6.

11. Composition contenant un polymère chloré et au moins un composé répondant à la formule IA :

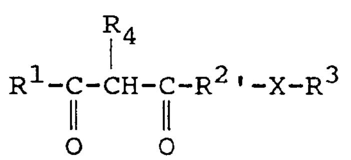

(IA)

dans laquelle $R^1$ et $R^3$ représentent chacun, indépendamment l'un de l'autre, un alkyle en $C_1$-$C_{12}$, un phényle, un phényle porteur d'un à trois alkyles en $C_1$-$C_{12}$, un phényl-alkyle en $C_7$-$C_{10}$ ou un phényl-alkyle en $C_7$-$C_{10}$ dont le cycle phényle porte d'un à trois alkyles en $C_1$-$C_{12}$, $R^1$ pouvant en outre représenter un radical -$R^{2'}$-X-$R^3$, $R^{2'}$ représente un alkylène en $C_1$-$C_{10}$, $R^4$ représente l'hydrogène, un alcoxy-carbonyle en $C_2$-$C_5$ ou un alcanoyle en $C_2$-$C_5$, et X représente l'oxygène ou le soufre.

12. Composition selon la revendication 11 dans laquelle X représente le soufre.

13. Composition selon la revendication 11 dans laquelle $R^{2'}$ représente un alkylène en $C_2$-$C_{10}$.

14. Composition selon la revendication 11 contenant en outre un composé époxydique et au moins un carboxylate le Me(II) et/ou un phénolate de Me(II), le symbole Me(II) représentant Ba, Ca, Mg, Cd ou Zn.

15. Composition selon la revendication 11 contenant en outre une huile de soja époxydée et au moins un carboxylate de Me(II), le symbole Me(II) représentant Ba, Ca, Mg ou Zn.

16. Composition selon la revendication 14 contenant en outre un phosphite.

17. Composition selon la revendication 11 caractérisée en ce que le polymère chloré est le poly-(chlorure

de vinyle).

18. Application de composés de formule IA selon la revendication 11 pour la stabilisation de polymères chlorés contre les effets destructeurs de la chaleur et/ou de la lumière.